# EUROPEAN PATENT SPECIFICATION

(11) **EP 2 158 197 B1**
(45) Date of publication and mention of the grant of the patent: **25.05.2011**
(21) Application number: 08773241.8
(22) Date of filing: 13.06.2008
(51) Int. Cl.: C07D 401/12

(54) **METHOD FOR THE PREPARATION OF HIGH PURITY ALMOTRIPTAN**
VERFAHREN ZUR HERSTELLUNG VON HOCHREINEM ALMOTRIPTAN
PROCÉDÉ DE PRÉPARATION D'ALMOTRIPTAN DE HAUTE PURETÉ

(30) Priority: 13.06.2007 CZ 20070408
(43) Date of publication of application: 03.03.2010
(73) Proprietor: Zentiva, k.s., Dolni Mecholupy 102 37 Praha 10 (CZ)
(72) Inventor: RIDVAN, Ludek, 102 00 Praha 10 (CZ); HRUBY, Petr, 182 00 Praha 8 (CZ); STACH, Jan, 190 16 Praha 9 (CZ); RADL, Stanislav, 250 84 Kvetnice (CZ); VOSLAR, Michal, 103 00 Praha-Kolovraty (CZ); BRUSOVA, Hana, 197 00 Praha 9 (CZ); KRUMBHOLCOVA, Lucie, 276 01 Melnik (CZ); ZATOPKOVA,Monika, 19800 Praha 9 (CZ)
(74) Representative: Jirotkova, Ivana
(86) International application number: PCT/CZ2008/000067
(87) International publication number: WO 2008/151584

(56) References cited:
- WO-A-94/02460
- WO-A-2006/129190
- WO-A-2007/013098
- US-A- 5 565 447
- BOSCH J ET AL: "Synthesis of 5-(sulfamoylmethyl)indoles" TETRAHEDRON, ELSEVIER SCIENCE PUBLISHERS, AMSTERDAM, NL, vol. 57, no. 6, 4 February 2001 (2001-02-04), pages 1041-1048, XP004316535 ISSN: 0040-4020 cited in the application

## Description

### Technical Field

The invention relates to a new method for the preparation and purification of 3-[2-(dimethylamino)ethyl]-5-(1-pyrrolidinylsulfonylmethyl)-1*H*-indole of formula (I) 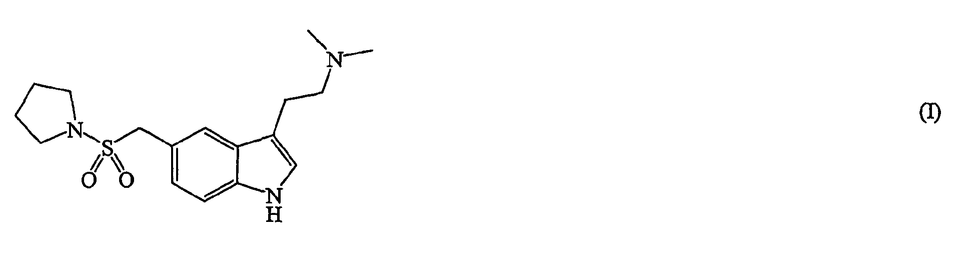 known under the generic name almotriptan.

### Background Art

Almotriptan is a selective agonist of 5-HT_{1b/1D} receptors and it is used to treat migraine. Preparation of almotriptan in the hydrochloride form is described in the EP patent 0 605 697 (Scheme 1).

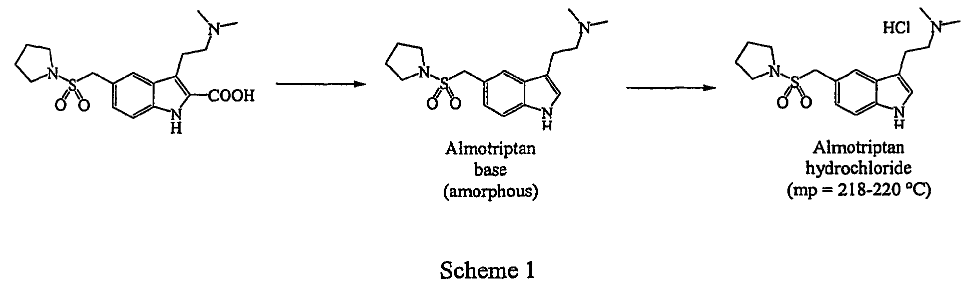

In a different way, almotriptan is prepared in the form of the crude amorphous base and subsequently also of D,L-malate in ES patent 2 084 560 (Scheme 2). The same procedure is also mentioned in Tetrahedron 57, 1041-1048, 2001.

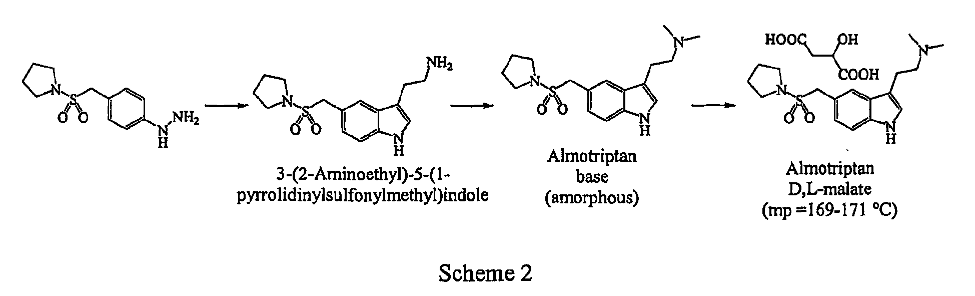

The intermediate of the synthesis, i.e. 3-(2-aminoethyl)-5-(1-pyrrolidinylsulfonylmethyl)-indole is isolated from the reaction mixture in the amorphous form and after dissolution it is used in the final synthetic step (*N-*methylation).

The above mentioned literature does not mention or comment on chemical purity of prepared almotriptan or its salts.

In patent application WO 2007/013098 crude almotriptan malate, prepared in accordance with the procedure shown in Scheme 2, is purified by conversion to a salt with 2- or 4-hydroxybenzoic acid, then almotriptan base is released, which is subsequently converted into D,L-malate (Scheme 3).

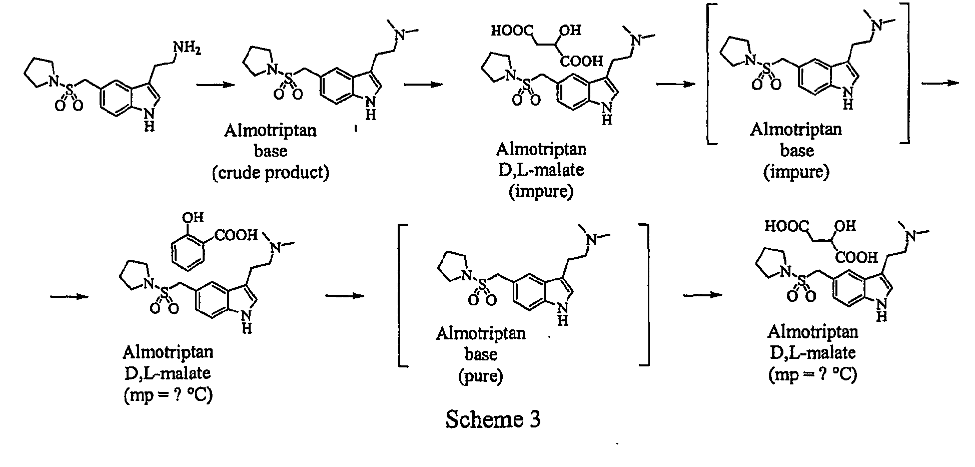

The authors do not mention any physical characteristics of the crystalline substances; they only mention the yields and HPLC purities. Besides the salts of almotriptan with 2-hydroxybenzoic acid and malic acids the authors also describe preparation of the oxalate in their examples.

It is obvious that the purification method of crude almotriptan is very important to achieve the required quality and yield of the substance.

The present invention brings a beneficial solution of preparation of highly pure almotriptan.

### Disclosure of Invention

The invention concerns a new method for the preparation and purification of the substance of formula (I), i.e. 3-[2-(dimethylamino)ethyl]-5-(1-pyrrolidinylsulfonylmethyl)-1*H*-indole 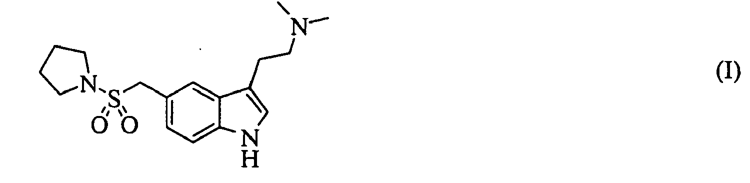 in which
(a) 4-(1-pyrrolidinylsulfonylmethyl)phenylhydrazine of formula (III) is converted by reaction with sodium or potassium 4-chloro-1-hydroxybutane-1-sulfonate to the salt of formula V, the temporarily generated hydrazonium salt of formula (VI) is converted into 3-(2-aminoethyl)-5-(1-pyrrolidinylsulfonylmethyl)indole of formula (VII), which is methylated in the last step without prior isolation by the action of a solution of formaldehyde and NaBH₄ to a solution of the almotriptan base, which provides a crystalline salt of almotriptan with an organic or inorganic acid;
(b) the salt of almotriptan is converted to the almotriptan base, which separates from the solution in the crystalline form;
(c) the almotriptan base is purified by crystallization from an organic solvent,
wherein the salt of formula (V) 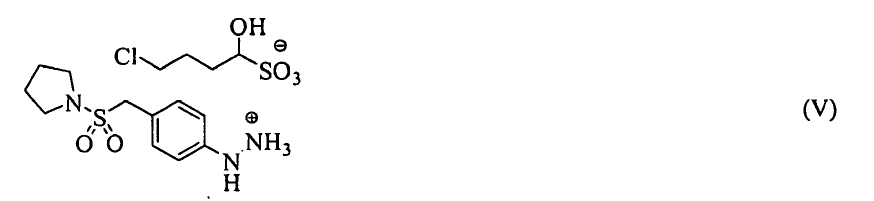 which is produced by mixing of 4-(1-pyrrolidinylsulfonylmethyl)phenylhydrazine hydrochloride and sodium or potassium 4-chloro-1-hydroxybutane-1-sulfonate, is isolated.

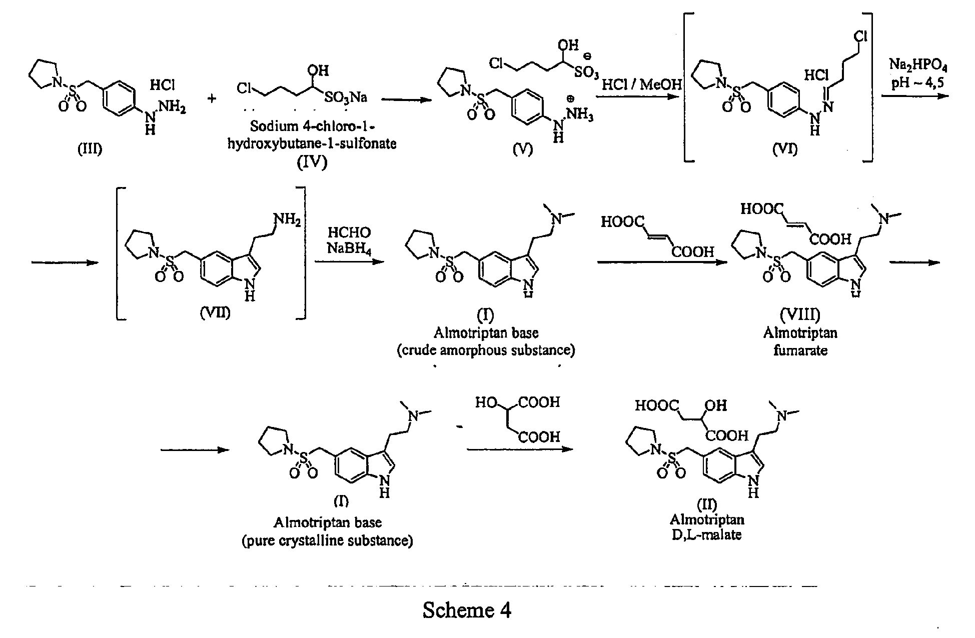

The invention also deals with the preparation of polymorphous forms of the crystalline base of almotriptan.

### Detailed Description of the Invention

As to (1) The starting 4-(1-pyrrolidinylsulfonylmethyl)phenylhydrazine (III) (Scheme 4) reacts with 4-chlorobutanal in the first step. With regard to instability of 4-chlorobutanale a derivative of this substance with the protected aldehydic group is commonly used, e.g. an acetal or adduct with NaHSO₃.

Arylhydrazines are normally prepared by reduction of the corresponding diazonium salt with tin dichloride. It is well known that arylhydrazine hydrochlorides prepared this way manifest a relatively high content of tin salts (see e.g. J. Am. Chem. Soc. 78, 5854-5856, 1956). The hydrazine (III) prepared by reduction of the corresponding diazonium salt with tin dichloride in accordance with the procedure published in Tetrahedron 57, 1041-1048, 2001, contains 13 to 16 % of tin, which corresponds roughly to 16 to 22 % of sulphate ash. Using such impure hydrazine hydrochloride (III) for the preparation of almotriptan is not convenient as the high content of tin salts considerably increases the consumption of Na₂HPO₄ in the adjustment of pH to the optimum value for conversion of hydrazine (III) to the indole derivative (VII). In addition, tin salts are not soluble at higher pH; the fine inorganic suspension is more difficult to stir and is very hard to filter.

Tin salts can be removed from crude hydrazine hydrochloride (III) by releasing of the hydrazine base, its extraction with an organic solvent and conversion to hydrochloride. However, this operation is rather time and material consuming.

Mixing of hydrazine hydrochloride (III) with sodium 4-chloro-1-hydroxybutane-1-sulfonate (IV) in water surprisingly causes very quick separation of a high yield of the crystalline salt, 4-(1-pyrrolidinylsulfonylmethyl)phenylhydrazine 4-chloro-1-hydroxybutane-1-sulfonate defined by formula (V), in high chemical purity; the inorganics remain dissolved in water after the formation of salt (V) and can be easily separated by filtering of the product. A similar salt is formed with other organic acids as well, e.g. p-toluenesulfonic or benzenesulfonic acids.

After dissolution of the salt (V) in an organic solvent, e.g. methanol, and addition of a strong acid, preferably hydrochloric acid, the salt (V) is decomposed, releasing 4-chlorobutanal, which together with the hydrazine (I) temporarily forms the hydrazone (VI). After adjustment of pH and several hours' reflux the indole derivative (VII) is obtained. ES patent 2 084 560, and an article in Tetrahedron 57, 1041-1048, 2001, describe this intermediate as a brown amorphous matter. However, the isolation is laborious and time and material consuming (evaporation of methanol, extraction with an organic solvent and its subsequent evaporation). Moreover, the unreacted 4-chorobutanal is also extracted with the crude intermediate (VII), which may react with the intermediate (VII), thus reducing the yield of almotriptan in the subsequent step and increasing the content of impurities at the same time.

We have surprisingly found out that if the intermediate (VII) is not isolated and a solution of formaldehyde and sodium tetrahydroborate is added to the cooled reaction mixture dropwise, crude almotriptan is obtained in a higher yield as slightly yellowish glass. The almotriptan base obtained this way is easily converted to a salt with a suitable acid, e.g. D,L-malate or the well-crystallizing fumarate. HPLC purity of thus prepared salt prepared is roughly 92%. Re-crystallization of these salts does not significantly increase the chemical purity.

As to (2) The almotriptan base is released from its salts in an aqueous environment by the effect of an aqueous solution of an inorganic or organic base that is stronger than almotriptan, such as alkali metal hydroxides and carbonates or ammonia, or triethylamine. The almotriptan base is extracted with a suitable organic solvent that is miscible with water to a limited extent. One can use e.g. ethyl acetate, ethylmethylketone, isobutylmethylketone, toluene or their mutual mixtures.

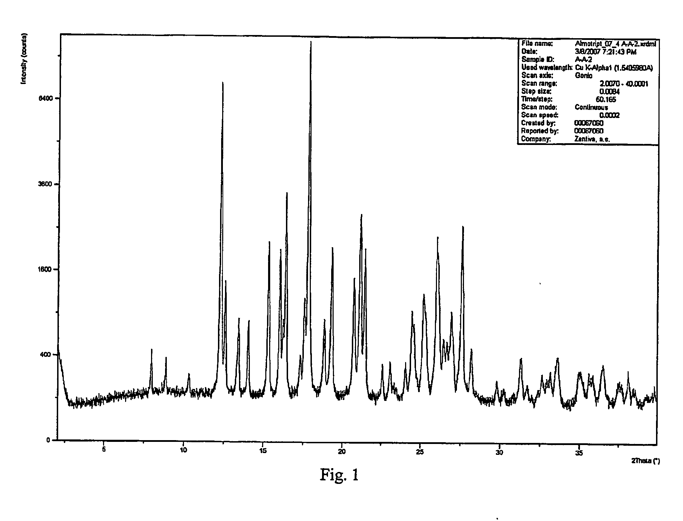

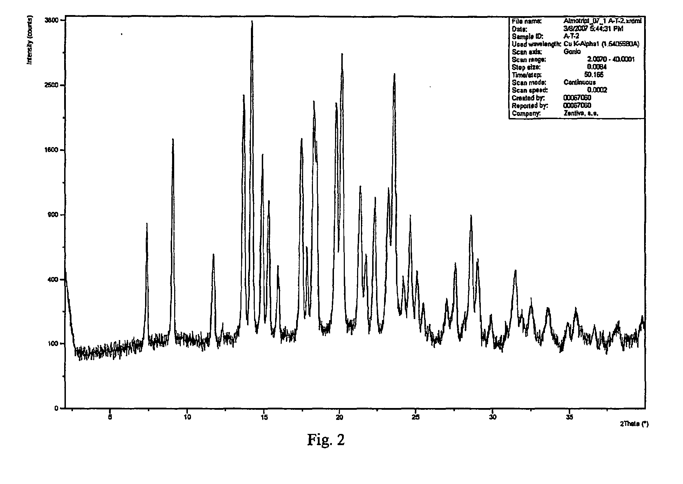

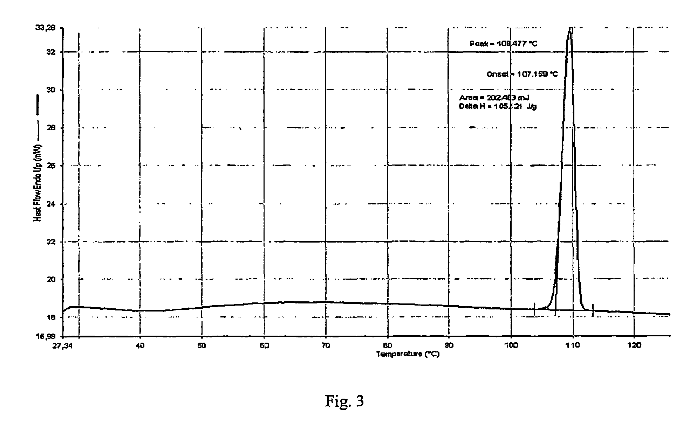

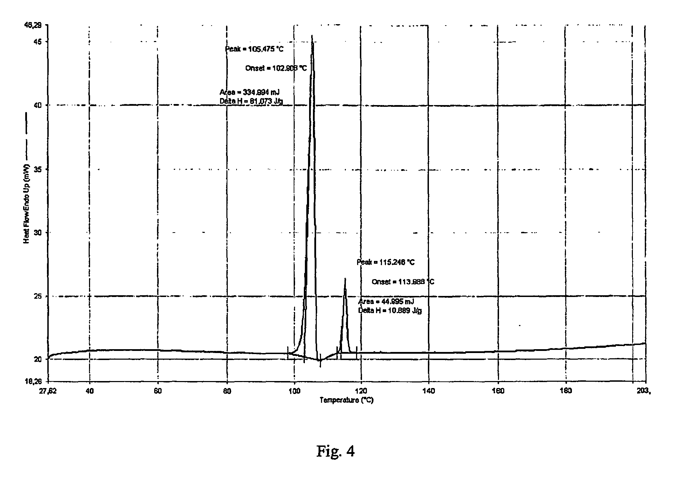

The almotriptan base released from the fumarate surprisingly easily crystallizes from a number of organic solvents. After a detailed analysis of the crystals we have found out that the almotriptan base crystallized in two crystal modifications depending on crystallization conditions and the used solvent (see Table 1). The thermodynamically more stable polymorph referred to as A (see the X-ray diffraction pattern in Fig. 1 and DSC record in Fig. 3) crystallizes from the same starting raw material with higher purity than the polymorph marked B (see the X-ray diffraction pattern in Fig. 2 and DSC record in Fig. 4).

**Table 1 Influence of the crystallization conditions of the almotriptan base on polymorphism and purity**

| **Exp. no.** | **Solvent** | **Almotriptan content** | **Cryst. form** | **RRT* 0.41** | **RRT 0.79** | **RRT 1.2** | **RRT 1.3** | **RRT 1.5** | **Sum of imp.** |
|---|---|---|---|---|---|---|---|---|---|
| Starting material | Toluene | 98.3 % | B | 0.31 % | 0.24 % | 0.51 % | 0.08 % | 0.22 % | 1.7 % |
| 1 | Toluene | 99.3 % | B | 0.06 % | 0.26 % | 0.16 % | 0.06 % | 0.06 % | 0.68 % |
| 2 | EtOAc | 99.0 % | B | 0.12 % | 0.23 % | 0.33 % | 0.08 % | 0.08 % | 1.0 % |
| 3 | 2-propanol | 99.4 % | B | 0.10 % | 0.21 % | 0.16 % | 0.07 % | 0.05 % | 0.60 % |
| 4 | Isobutyl-methylketone | 99.2 % | B | 0.07 % | 0.24 % | 0.17 % | 0.05 % | 0.05 % | 0.78 % |
| 5 | Dioxan | 99.7 % | A | - | 0.12 % | 0.05 % | - | - | 0.27 % |
| 6 | Acetone | 99.7 % | A | - | 0.04 % | 0.11 % | - | - | 0.29 % |
| 7 | Butanone | 99.5 % | A | - | 0.16 % | 0.18 % | - | - | 0.40 % |
| 8 | Acetonitrile | 99.7 % | A | - | 0.18 % | 0.06 % | - | - | 0.29 % |
| 9 | THF | 99.8% | A | - | 0.14% | - | - | - | 0.21% |
| 10 | EtOH | 99.6 % | A | - | 0.16 % | 0.11 % | - | - | 0.36 % |

| | | | | | | | | | |
|---|---|---|---|---|---|---|---|---|---|
| ***RRT** = Relative retention time with respect to almotriptan in evaluation of HPLC purity | | | | | | | | | |

Table 2 summarizes the characteristic peaks for polymorphs A and B. The diffraction records were obtained through measurements with an X'PERT PRO MPD PANalytical laboratory X-ray diffractometer in the θ - θ reflection mode with a CuKα (λ=1.5402Å, 45kV/40mA) copper X-ray tube, with graphite monochromator, in the range of 2 - 40° 2θ with the step of 0.01° 2θ.

**Table 2 Characteristic peaks corresponding to the crystalline form A and form B of the almotriptan base**

| 2theta | d | Irel | | 2theta | d | Irel |
|---|---|---|---|---|---|---|
| 7.97 | 11.079 | 3.34 | | 7.40 | 11.935 | 19.25 |
| 8.88 | 9.947 | 2.49 | | 9.08 | 9.727 | 44.25 |
| 12.3 | 7.192 | 76.87 | | 13.67 | 6.471 | 60.68 |
| 15.29 | 5.789 | 23.55 | | 14.19 | 6.238 | 100 |
| 16.38 | 5.407 | 39.44 | | 14.86 | 5.957 | 39.43 |
| 17.84 | 4.967 | 100 | | 15.28 | 5.795 | 24.89 |
| 19.31 | 4.593 | 21.98 | | 17.44 | 5.082 | 45.69 |
| 21.14 | 4.2 | 32.23 | | 18.28 | 4.849 | 57.53 |
| 21.43 | 4.144 | 23.33 | | 19.78 | 4.485 | 59.11 |
| 26.04 | 3.419 | 23.28 | | 20.16 | 4.402 | 82.12 |
| 27.59 | 3.23 | 29.8 | | 21.35 | 4.158 | 28.98 |
| | | | | 22.31 | 3.981 | 25.8 |
| | | | | 23.61 | 3.766 | 73.31 |
| | | | | 24.66 | 3.607 | 19.81 |
| | | | | 28.58 | 3.121 | 21.33 |

Characteristic DSC records corresponding to the forms A and B are presented in Figures 3 and 4. The DSC records were obtained by measurements with the Perkin Elmer Pyris 1 DSC instrument at the heating rate of 10 °C/min in a nitrogen stream.

As suitable solvents for crystallization one can use e.g. aromatic hydrocarbons (e.g. toluene), lower ketones (e.g. acetone, butanone or isobutylmethylketone), C₁-C₄ lower alcohols (e.g. ethanol or 2-propanol), acetic acid esters (e.g. methyl, ethyl or isopropyl esters), cyclic ethers (e.g. tetrahydrofuran or dioxan), or acetonitrile and their mutual mixtures.

The purified almotriptan base is then converted to a pharmaceutically acceptable salt, preferably D,L-malate of formula (II).

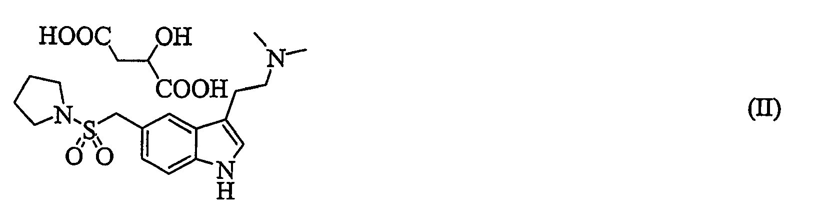

### Brief Description of Drawings

Figure 1 represents an X-ray record characteristic for the crystalline form A of the almotriptan base.
Figure 2 represents an X-ray record characteristic for the crystalline form B of the almotriptan base.
Figure 3 represents a DSC record characteristic for the crystalline form A of the almotriptan base.
Figure 4 represents a DSC record characteristic for the crystalline form B of the almotriptan base.

The invention is described in a more detailed way in the following examples.

### Examples

### Example 1: Preparation of 4-(1-pyrrolidinylsulfonylmethyl)phenylhydrazine 4-chloro-1-hydroxybutane-1-sulfonate (V)

Solid sodium 4-chloro-1-hydroxybutane-1-sulfonate (IV) (21 g) is added to a solution of 4-(1-pyrrolidinylsulfonylmethyl)phenylhydrazine (III) (33 g, HPLC purity 89 %, tin content 13 %, sulphate ash 20 %) in water (80 ml). After 15 minutes' stirring at the laboratory temperature the mixture is cooled to 0 °C, the separated salt (V) is filtered and washed with iced water.

Yield: 31 g (89 % - related to pure hydrazine (III), HPLC purity 96 %, sulphate ash 4 %).

### Example 2: Preparation of 4-(1-pyrrolidinylsulfonylmethyl)phenylhydrazine p-toluenesulfonate (IX)

Solid *p*-toluenesulfonic acid (51 g) is added to a solution of 4-(1-pyrrolidinyl-sulfonylmethyl)phenylhydrazine (III) (100 g, HPLC purity 89 %, tin content 13 %, sulphate ash 20 %) in water (200 ml). After 15 minutes' stirring at the laboratory temperature the mixture is cooled to 0 °C, the separated salt is filtered and washed with iced water.

Yield: 93 g (91 % - related to pure hydrazine (III), HPLC purity 95 %, sulphate ash 6 %).

### Example 3: Preparation of almotriptan fumarate (VIII)

Concentrated hydrochloric acid is added dropwise to a solution of 4-(1-pyrrolidinyl-sulfonylinethyl)phenylhydrazine 4-chlor-1-hydroxybutane-1-sulfonate (V) (31 g) in MeOH (300 ml) at the temperature of 20-25 °C and the solution is stirred for 30 minutes. By addition of a solution of Na₂HPO₄ (30 g in 90 ml of water) pH is adjusted to the value of 4.6 to 4.9 and the mixture is refluxed for 4 hours. After cooling activated charcoal is added and the mixture is filtered through diatomite. By addition of 10% aqueous Na₂CO₃ (ca. 70 ml) pH is adjusted to the value of 7.0 to 7.5. A solution of 35% aqueous formaldehyde (70 ml in 70 ml of methanol) and sodium tetrahydroborate (10 g in 140 ml of water) are added dropwise at the same time to the reaction mixture at the temperature of 10-15 °C during 1 hour. The mixture is then stirred for another 20 to 30 min at the temperature of 10-15°C. The reaction mixture is acidified by adding concentrated hydrochloric acid dropwise until pH 5.0 to 5.5. 1 g of activated charcoal is added and the mixture is filtered through diatomite for 10 minutes. Then, pH of the solution is adjusted to 8 to 9 by addition of 35% free ammonia. After that, MeOH is removed by distillation and the product is extracted with ethyl acetate (3 x 100 ml). The solution of crude almotriptan is concentrated under reduced pressure. The evaporation residue is dissolved in 2-propanol (20 ml) and added dropwise to a solution of fumaric acid (4 g) in 2-propanol (100 ml) heated to up to 45-50 °C. The mixture is cooled to 30 °C during one hour and stirred at this temperature for 1 hour. The separated crystals are filtered and washed with 2-propanol.

Yield: 11.5 g (38 %), HPLC purity 91.8 %, melting temp. = 206-210 °C.

### Example 4: Preparation of 1-hydroxymethyl-3-[2-(dimethylamino)ethyl]-5-(1-pyrrolidinylsulfonylmethyl)-1H-indole fumarate (X)

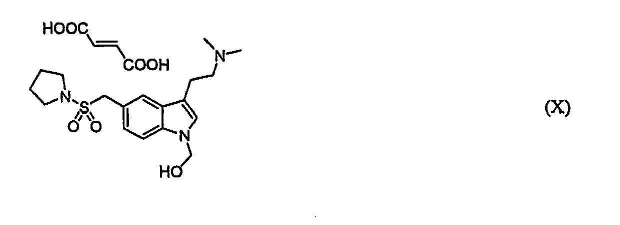

Concentrated hydrochloric acid is added dropwise to a solution of 4-(1-pyrrolidinylsulfonylinethyl)phenylhydrazine 4-chloro-1-hydroxybutane-1-sulfonate (V) (31 g) in MeOH (300 ml) at the temperature of 20-25 °C and the solution is stirred for 30 minutes. By addition of a solution of Na₂HPO₄ (30 g in 90 ml of water) pH is adjusted to the value of 4.6 to 4.9 and the mixture is refluxed for 4 hours. After cooling activated charcoal is added and the mixture is filtered through diatomite. By addition of 10% aqueous Na₂CO₃ (ca. 70 ml) pH is adjusted to the value of 7.0 to 7.5. A solution of 35% aqueous formaldehyde (140 ml in 70 ml of methanol) and sodium tetrahydroborate (10 g in 140 ml of water) is added dropwise at the same time to the reaction mixture at the temperature of 10-15°C during 1 hour. The mixture is then stirred at the temperature of 30 °C for another 6 hours. 1 g of activated charcoal is added and after 10 mins the mixture is filtered through diatomite. Then, pH of the solution is adjusted to ca. 9 by addition of 10% aqueous Na₂CO₃. After that, MeOH is removed by distillation and the product is extracted with ethyl acetate (3 x 100 ml). The solution of the crude product is concentrated under reduced pressure. The evaporation residue is dissolved in ethanol (20 ml) and added dropwise to a solution of fumaric acid (4 g) in ethanol (80 ml). The mixture is stirred at the laboratory temperature for 1 hour. The separated crystals are filtered and washed with ethanol.

Yield: 14 g (30 %), HPLC purity 93.4 %, melting temperature = 72-80 °C.

Re-crystallization from methanol produces a salt with the base - acid ratio of 2:1, melting temp. =186-193 °C.

### Example 5: Preparation of almotriptan fumarate (VIII)

1-Hydroxymethyl-3-[2-(dimethylamino)ethyl]-5-(1-pyrrolidinylsulfonylmethyl)-1*H-*indole fumarate (10 g) is stirred in 10% aqueous Na₂CO₃ (100 ml) and the free base is extracted with ethyl acetate. The extract is dried and concentrated under reduced pressure. The evaporation residue is dissolved in methanol (60 ml), 30% ammonia solution (20 ml) is added to the solution and the mixture is stirred at the laboratory temperature for 8 hours. Methanol is evaporated; the free almotriptan base is extracted with ethyl acetate. The solution of the product is concentrated under reduced pressure. The evaporation residue is dissolved in ethanol (10 ml) and added dropwise to a solution of fumaric acid (2.4 g) in ethanol (40 ml). The mixture is stirred at the laboratory temperature for 1 hour. The separated crystals are filtered and washed with ethanol.

Yield: 7.4 g (80 %), HPLC purity 91.7 %, melting temp. = 207-211 °C.

### Example 6: Preparation of the crystalline base of almotriptan (I) - polymorph A

Almotriptan fumarate (22 g, HPLC purity 91.6 %) is stirred in a mixture of 10% aqueous Na₂CO₃ (150 ml) and ethyl acetate (100 ml). The organic phase is separated, dried and concentrated by evaporation of the solvent under reduced pressure. The evaporation residue is dissolved in acetone (15 ml). After inoculation the mixture is stirred at the temperature of 10 °C for 2 hours. The separated crystals are aspirated and washed with cold acetone.

Yield: 10.5 g (66 %), HPLC purity 99.2 %, melting temp. =109-110 °C.

### Example 7: Preparation of the crystalline base of almotriptan (I) - polymorph A

The almotriptan base (10 g, HPLC purity 99.2 %) is dissolved in butanone (20 ml) at the temperature of 80 °C. The solution is cooled to the temperature of 40 to 50 °C, inoculated and left to slowly cool down to the temperature of 20 °C. The separated crystals are aspirated and washed with cold butanone.

Yield: 7.5 g (75 %), HPLC purity 99.7 %, melting temp. =109-111 °C.

### Example 8: Preparation of the crystalline base of almotriptan (I) - polymorph B

Almotriptan fumarate (22 g, HPLC purity 91.6 %) is stirred in a mixture of 10% aqueous Na₂CO₃ (150 ml) and ethyl acetate (100 ml). The organic phase is separated, dried and concentrated by evaporation of the solvent under reduced pressure. The evaporation residue is dissolved in toluene (30 ml). After inoculation the mixture is stirred at the temperature of 20 °C for 2 hours. The separated crystals are aspirated and washed with toluene.

Yield: 13.1 g (81 %), HPLC purity 97.6 %, melting temp. =104-106 °C.

### Example 9: Preparation of the crystalline base of almotriptan (I) - polymorph A

Almotriptan fumarate (22 g, HPLC purity 91.6 %) is stirred in a mixture of 10% aqueous Na₂CO₃ (150 ml) and ethyl acetate (100 ml). The organic phase is separated, dried and concentrated by evaporation of the solvent under reduced pressure. The evaporation residue is dissolved in ethanol (20 ml). After inoculation the mixture is stirred at the temperature of 10 °C for 2 hours. The separated crystals are aspirated and washed with cold ethanol.

Yield: 11.7 g (72 %), HPLC purity 99.6 %, melting temp. =110-111 °C.

### Example 10: Preparation of almotriptan D,L-malate (II)

The almotriptan base (10 g, HPLC purity 99.7 %) is dissolved in MeOH (15 ml) at the temperature of 40°C and added dropwise to a solution of D,L-malic acid (4 g) in MeOH (30 ml), heated up to 40 °C. The mixture is left to slowly cool down to 20 °C and is stirred for another hour. The separated crystals are filtered and washed with MeOH.

Yield: 13 g (93 %), HPLC purity 99.9 %, melting temp. =169-171 °C.

### Evacuation of chemical purity

**Column**: Gemini C18 110A (150 x 4.6 cm, 3 µm)
**Column temp.**: 30°C
**Mobile phase**: A: 0.01M phosphate buffer (pH 10); B: methanol
**Elution**:

| Time | A | B |
|---|---|---|
| 0 | 80 | 20 |
| 2 | 80 | 20 |
| 25 | 30 | 70 |
| 35 | 30 | 70 |
| 36 | 80 | 20 |
| 40 | 80 | 20 |

**Flow rate**: 1.0 ml/min
**Detection**: UV 227 nm
**Detection limit**: 0.01 %

## Claims

1. A method for the preparation and purification of 3-[2-(dimethylamino)ethyl]-5-(1-pyrrolidinylsulfonylmethyl)-1*H-*indole of formula (I) 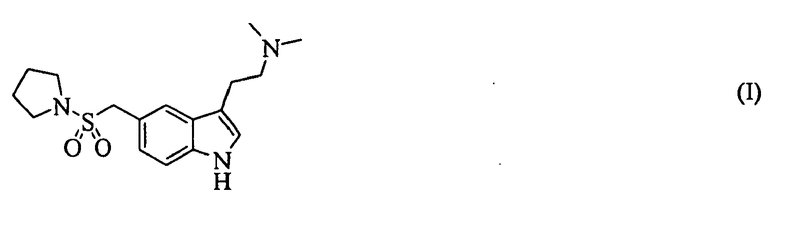 **characterized in that**
(a) 4-(1-pyrrolidinylsulfonylmethyl)phenylhydrazine is converted by reaction with sodium or potassium 4-chloro-1-hydroxybutane-1-sulfonate to the salt of formula V 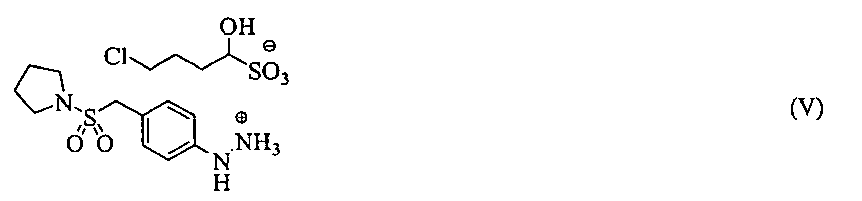 the temporarily generated hydrazonium salt of formula (VI) is converted to 3-(2-aminoethyl)-5-(1-pyrrolidinylsulfonylmethyl)indole, which is methylated in the last step without prior isolation by the action of a solution of formaldehyde and NaBH₄ to a solution of the almotriptan base, which provides a crystalline salt of almotriptan with an organic or inorganic acid;
(b) the salt of almotriptan is converted to the almotriptan base, which separates from the solution in the crystalline form;
(c) the almotriptan base is purified by crystallization from an organic solvent,
wherein the salt of formula (V) 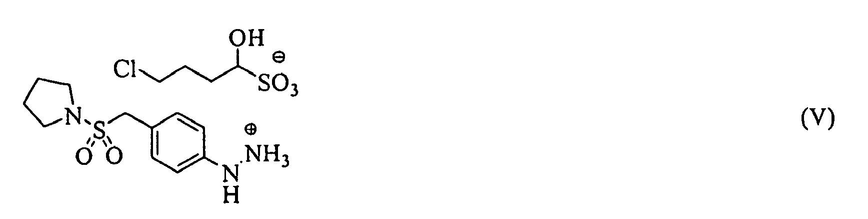 which is produced by mixing of 4-(1-pyrrolidinylsulfonylmethyl)phenylhydrazine hydrochloride and sodium or potassium 4-chloro-1-hydroxybutane-1-sulfonate, is isolated.

2. The method according to claim 1, **characterized in that** in step (a) the crude almotriptan base is converted by the action of fumaric acid to its salt in an organic solvent.

3. The method according to claim 2, **characterized in that** the solvent is selected from methanol, ethanol, n-butanol or 2-propanol.

4. The method according to claim 1, **characterized in that** in step (b) the almotriptan salt according to claim 1 is treated with an aqueous solution of a base selected from KOH, NaOH, K₂CO₃, Na₂CO₃ or ammonia, whereupon the almotriptan base is extracted with an organic solvent selected from ethyl acetate, ethylmethylketone, isobutylmethylketone, toluene or their mutual mixtures.

5. The method according to claim 4, **characterized in that** the almotriptan base is crystallized from the organic solvent.

6. The method according to claim 5, **characterized in that** the crystallization is achieved by concentrating, by cooling the solution, or by combination of both.

7. The method according to claim 1, **characterized in that** in step (b) the almotriptan base of formula (I) is re-crystallized from a solvent selected from aromatic hydrocarbons, ketones, alcohols, ethers, esters and acetic acid nitrile.

8. The method according to claim 7, **characterized in that** the solvent for crystallization of the almotriptan base of formula (I) is selected from toluene, acetone, butanone, isobutylmethylketone, acetonitrile, methanol, ethanol, 2-propanol, n-butanol, tetrahydrofuran, dioxan and methyl, ethyl or isopropyl esters of acetic acid.

9. 4-(1-Pyrrolidinylsulfonylmethyl)phenylhydrazine 4-chloro-1-hydroxybutane-1-sulfonate defined by formula (V)

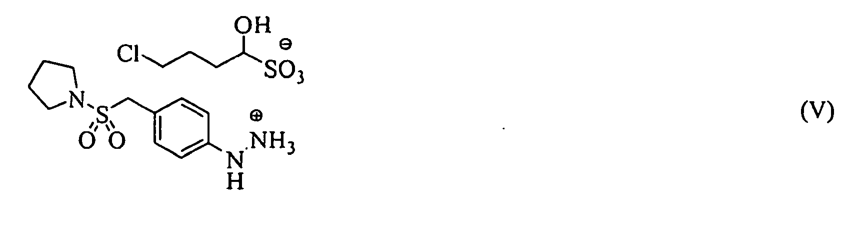

## Patentansprüche

1. Verfahren zur Herstellung und Reinigung von 3-[2-(Dimethylamino)ethyl]-5-(1-pyrrolidinylsulfonyl-methyl)-1H-indol der Formel (I): 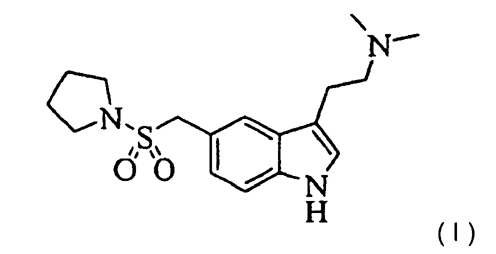 **dadurch gekennzeichnet, dass**
(a) 4-(1-Pyrrolidinylsulfonylmethyl)phenyl-hydrazin durch Umsetzen mit Natrium- oder Kalium-4-chlor-1-hydroxybutan-1-sulfonat zu einem Salz der Formel (V) umgewandelt wird: 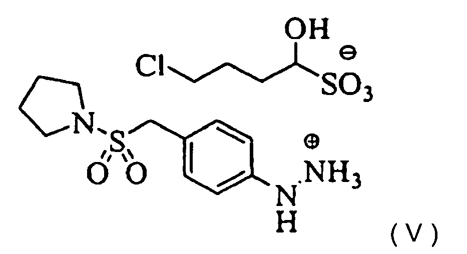 das zunächst erzeugte Hydrazoniumsalz der Formel (VI) zu 3-(2-Aminoethyl)-5-(1-pyrrolidinylsulfonylmethyl)-indol umgewandelt wird, das im letzten Schritt ohne vorherige Isolierung durch die Einwirkung einer Lösung von Formaldehyd und NaBH₄ zu einer Lösung der Almotriptan-Base methyliert wird, wodurch ein kristallines Salz von Almotriptan mit einer organischen oder anorganischen Säure bereitgestellt wird;
(b) das Salz von Almotriptan in die Almotriptan-Base umgewandelt wird, die sich von der Lösung in kristalliner Form trennt;
(c) die Almotriptan-Base durch Kristallisation aus einem organischen Lösungsmittel gereinigt wird,
wobei das Salz der Formel (V) 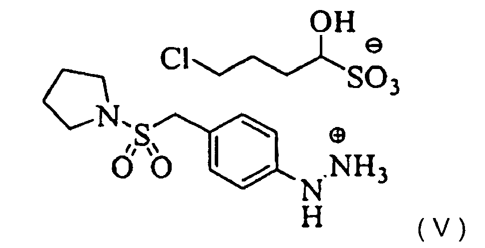 das durch Mischen von 4-(1-Pyrrolidinylsulfonyl-methyl)phenylhydrazin-Hydrochlorid und Natrium- oder Kalium-4-chlor-1-hydroxybutan-1-sulfonat hergestellt wird, isoliert wird.

2. Verfahren gemäss Anspruch 1, **dadurch gekennzeichnet, dass** in Schritt (a) die rohe Almotriptan-Base durch die Einwirkung von Fumarsäure in einem organischen Lösungsmittel zu seinem Salz umgewandelt wird.

3. Verfahren gemäss Anspruch 2, **dadurch gekennzeichnet, dass** das Lösungsmittel aus Methanol, Ethanol, n-Butanol oder 2-Propanol ausgewählt ist.

4. Verfahren gemäss Anspruch 1, **dadurch gekennzeichnet, dass** in Schritt (b) das Almotriptansalz gemäss Anspruch 1 mit einer wässrigen Lösung einer Base, ausgewählt aus KOH, NaOH, K₂CO₃, Na₂CO₃ oder Ammoniak, behandelt wird, worauf die Almotriptan-Base mit einem organischen Lösungsmittel, ausgewählt aus Ethylacetat, Ethylmethylketon, Isobutylmethylketon, Toluol oder deren gegenseitigen Mischungen, extrahiert wird.

5. Verfahren gemäss Anspruch 4, **dadurch gekennzeichnet, dass** die Almotriptan-Base aus einem organischen Lösungsmittel kristallisiert wird.

6. Verfahren gemäss Anspruch 5, **dadurch gekennzeichnet, dass** die Kristallisation durch Konzentration, durch Kühlen der Lösung oder durch eine Kombination von beidem erreicht wird.

7. Verfahren gemäss Anspruch 1, **dadurch gekennzeichnet, dass** in Schritt (b) die Almotriptan-Base der Formel (I) aus einem Lösungsmittel, ausgewählt aus aromatischen Kohlenwasserstoffen, Ketonen, Alkoholen, Ethern, Estern und Essigsäurenitril, umkristallisiert wird.

8. Verfahren gemäss Anspruch 7, **dadurch gekennzeichnet, dass** das Lösungsmittel zur Kristallisation der Almotriptan-Base der Formel (I) aus Toluol, Aceton, Butanon, Isobutylmethylketon, Acetonitril, Methanol, Ethanol, 2-Propanol, n-Butanol, Tetrahydrofuran, Dioxan und Methyl-, Ethyl- oder Isopropylestern von Essigsäure ausgewählt wird.

9. 4-(1-Pyrrolidinylsulfonylmethyl)phenylhydrazin-4-chlor-1-hydroxybutan-1-sulfonat, definiert durch die Formel (V):

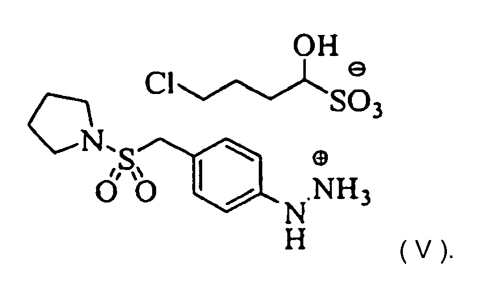

## Revendications

1. Un procédé pour la préparation et la purification de 3-[2-(diméthylamino)-éthyl]-5-(1-pyrrolidinylsulfonylméthyl)-1H-indole de formule (I) 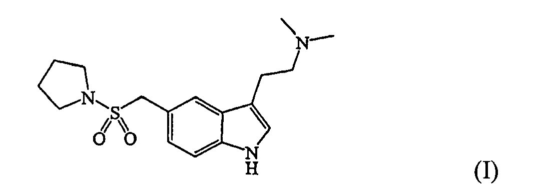 **caractérisé en ce que**
(a) de la 4-(1-pyrrolidinylsulfonylméthyl)-phénylhydrazine est convertie par réaction avec du 4-chloro-1-hydroxybutane-1-sulfonate de sodium ou de potassium en sel de formule V 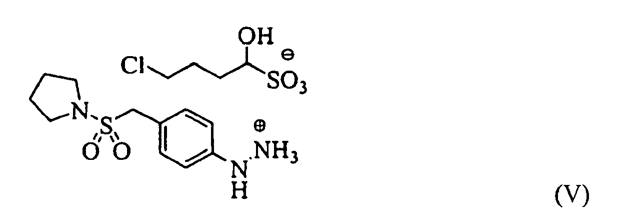 le sel d'hydrazonium de formule (VI) temporairement généré est converti en 3-(2-aminoéthyl)-5-(1-pyrrolidinylsulfonylméthyl)-indole, lequel, dans la dernière étape, est méthylé, sans isolation préalable, par action d'une solution de formaldéhyde et de NaBH₄ en une solution d'almotriptan, base, ce qui conduit à l'obtention d'un sel cristallin d'almotriptan et d'un acide organique ou inorganique;
(b) le sel d'almotriptan est converti en almotriptan base qui se sépare, sous forme cristalline, de la solution;
(c) l'almotriptan base est purifié par cristallisation dans un solvant organique,
dans lequel est isolé le sel de formule (V) 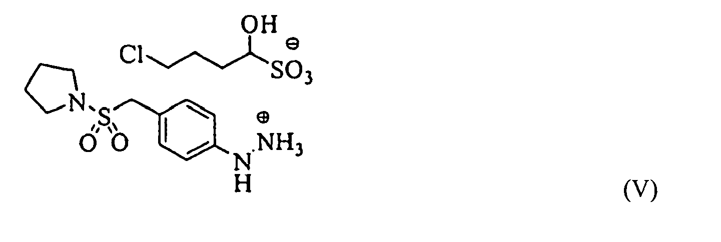 qui est produit par mélange de chlorhydrate de 4-(1-pyrrolidinyl-sulfonylméthyl)-phénylhydrazine et de 4-chloro-1-hydroxy-butane-1-sulfonate de sodium ou de potassium.

2. Le procédé selon la revendication 1, **caractérisé en ce que** dans l'étape (a) l'almotriptan base brut est converti en son sel, dans un solvant organique, par action de l'acide fumarique.

3. Le procédé selon la revendication 2, **caractérisé en ce que** le solvant est choisi dans le groupe comprenant le méthanol, l'éthanol, le n-butanol ou le 2-propanol.

4. Le procédé selon la revendication 1, **caractérisé en ce que** dans l'étape (b) le sel d'almotriptan selon la revendication 1, est traité par une solution aqueuse d'une base choisie dans le groupe comprenant KOH, NaOH, K₂CO₃, Na₂CO₃ ou l'ammoniaque, après quoi l'almotriptan base est extrait par un solvant organique choisi parmi l'acétate d'éthyle, l'éthylméthylcétone, l'isobutylméthylcétone, le toluène ou leurs mélanges entre eux.

5. Le procédé selon la revendication 4, **caractérisé en ce que** l'almotriptan base cristallise dans un solvant organique.

6. Le procédé selon la revendication 5, **caractérisé en ce que** la cristallisation est obtenue par concentration ou refroidissemant de la solution, ou par la combinaison des deux.

7. Le procédé selon la revendication 1, **caractérisé en ce que** dans l'étape (b) l'almotriptan base de formule (1) est re-cristallisé dans un solvant choisi dans le groupe comprenant les hydrocarbures aromatiques, les cétones, les alcools, les éthers, les esters, et le nitrile d'acide acétique.

8. Le procédé selon la revendication 7, **caractérisé en ce que** le solvant pour la cristallisation de l'almotriptan base de formule (I) est choisi parmi le toluène, l'acétone, le butanone, l'isobutylméthylcétone, l'acétonitrile, le méthanol, l'éthanol, le 2-propanol, le n-butanol, le tétrahydrofuranne, le dioxanne et les esters méthyliques, éthyliques ou isopropyliques de l'acide acétique.

9. 4-(1-pyrrolidinylsulfonyl-méthyl)-phénylhydrazine 4-chloro-1-hydroxybutane-1-sulfonate défini par la formule (V).
